# EUROPEAN PATENT SPECIFICATION

(11) **EP 1 235 720 B1**
(45) Date of publication and mention of the grant of the patent: **12.10.2005**
(21) Application number: 00977750.9
(22) Date of filing: 01.12.2000
(51) Int. Cl.: B65D 47/10, A61M 5/32, A61M 5/50, B29C 44/04

(54) **CLOSURE ASSEMBLY IN PARTICULAR FOR HYPODERMIC SYRINGES**
VERSCHLUSSANORDNUNG, INSBESONDERE FÜR SUBKUTANINJEKTIONSSPRITZEN
ENSEMBLE DE FERMETURE, NOTAMMENT DESTINE A DES SERINGUES HYPODERMIQUES

(30) Priority: 08.12.1999 GB 9928884
(43) Date of publication of application: 04.09.2002
(73) Proprietor: NMT Group PLC, Scotland EH53 0TH (GB)
(72) Inventor: TARGELL, John, Ayrshire, Central Scotland KA1 5NH (GB)
(74) Representative: Warren, Keith Stanley
(86) International application number: PCT/GB2000/004573
(87) International publication number: WO 2001/042104

(56) References cited:
- EP-A- 0 300 694
- EP-A- 0 413 414
- EP-A- 0 747 075
- EP-A- 0 753 323
- EP-A- 0 911 132
- WO-A-98/13077
- WO-A-98/48869
- FR-A- 2 755 614
- US-A- 4 446 984
- US-A- 5 084 018
- US-A- 5 385 551
- US-A- 5 902 276
- US-A- 5 997 512
- US-A- 6 123 688

## Description

This invention relates to a closure assembly for use in providing a seal, especially a hermetic seal, the closure assembly comprising a portion which can be broken away to provide an aperture.

An important, though by no means exclusive, application for such closure assemblies is in a hypodermic syringe of the kind which provides for retraction of the needle after use into the body of the plunger. To this end, the forward end wall of the plunger can be provided with a so-called "bursting disc" which breaks away at the end of the injection stroke to provide an aperture through which the needle may pass into the body of the plunger. One form of such syringe is described in our prior EP-A-776225.

Typically the plunger is moulded from a plastics material and the "bursting disc" is defined by a circular line of weakness in the forward end wall of the plunger. To ensure fracture at the line of weakness, it is necessary to mould the "bursting disc" component from a brittle material such as polystyrene which imposes restrictions on the range of materials that can be used.

According to one aspect, the present invention consists in a device comprising a rim portion defining an aperture and a removable blocking portion which closes the aperture, said portions being united in sealing contact with each other at a moulding interface which extends around the blocking portion, both portions being produced as plastics mouldings, one portion having been produced in advance of the other so that moulding of the other portion can be effected with said one portion *in situ*, the blocking portion being irreversibly removable from the rim portion that, once the two portions have been separated from one another, they cannot be restored to the same condition of sealing and separation resistance simply by re-inserting the blocking portion into the rim portion ,

The arrangement is preferably such that the two portions are united together by the moulding process to afford a well-defined threshold at which the blocking portion breaks away from the rim portion in response to the application of mechanical force to the blocking portion.

Preferably the arrangement is such that the blocking portion breaks away cleanly from the rim portion at said interface to leave a dimensionally well-defined aperture.

Preferably one of the portions is produced as a plastics moulding in a mould having moulding surfaces defined, in part, by the other portion so as to provide said moulding interface.

The two portions may be of plastics materials having different chemical compositions and/or characteristics or they may both be of the same plastics material.

The blocking portion may be capable of being broken away from the rim portion without deformation of either of the two portions and, to this end, said interface may be shaped so that no deformation of either part occurs when breaking away the blocking portion by loading the same relative to the rim portion in at least one direction.

Alternatively, the two portions may be interlocked with each other so that some deformation of at least one of the parts occurs when breaking away the blocking portion.

Preferably at the moulding interface, an annular surface of one of said portions is conformed with an annular surface of the other portion in the course of moulding said one portion.

The annular surfaces may be cylindrical or of other configuration, e.g. conical.

At the moulding interface, the portions may intimately contact each other with a degree of fusion bonding consistent with securing fracture preferentially at the interface region when the blocking portion is caused to break away from the rim portion.

The device preferably includes means for resisting removal of the blocking portion so that the blocking portion can only be separated from the rim portion by application of axial load of predetermined magnitude.

Such means may comprise a detent arrangement acting between the portions. The detent arrangement may be provided at or in the vicinity of said moulding interface. For example, the rim and blocking portions may, by virtue of the mould design, have interengaging elements which lock the portions together to prevent separation of the portions, at least one of the elements being resiliently deformable to allow the disengagement of the elements on application of sufficient loading to the blocking portion relative to the rim portion.

Resistance to separation of the portions may additionally or alternatively be afforded by the nature of the interaction between the rim and blocking portions at the moulding interface. For instance, there may a shrink type fit between the portions at the moulding interface obtained by material shrinkage during cooling following the moulding process.

As a further addition or alternative to the detent arrangement and/or shrink type fit mentioned above, resistance to separation may be provided by fusion bonding between said portions at the moulding interface. Such fusion bonding may range from relatively weak, e.g. as a result of some degree of diffusion of material from one portion across the moulding interface into the other portion, through to relatively strong, including for example significant welding together of the portions at the moulding interface.

Depending on the nature of interaction desired between the rim and blocking portions at the moulding interface, the material or materials from which said portions are produced may be selected so as to secure the desired extent of fusion bonding, if any. For example, if negligible fusion bonding is desired, the materials employed will usually differ in chemical and/or physical characteristics and will be such that no significant diffusion of material takes place across the moulding interface as a result of the moulding process. Similarly the materials can be appropriately selected to obtain the desired degree of fusion bonding where there is a requirement to develop a fusion bond at the moulding interface.

In the latter case for example, the rim and blocking portions may be composed of the same material so that significant fusion of the material takes place between the two portions.

The width of the moulding interface and hence the zone of contact between the two portions may be selected with regard to the required effectiveness of the seal and/or with regard to the resistance to separation of the blocking portion required at said interface. For instance, a given degree of resistance may be secured by a relatively narrow interface where the portions are strongly fusion bonded together whereas a wider interface may be required if resistance is afforded by shrinkage and/or a weak fusion bond.

Preferably the closure device is produced by a two-shot moulding process in which one portion, e.g. the blocking portion, is initially formed and located so that an annular surface thereof forms a boundary surface of the mould cavity in which the other portion is produced so that, during formation of the second portion, an annular surface of the latter is conformed with the annular surface of the first portion.

Because the closure device of the present invention is not limited to fabrication using brittle materials such as styrene, a wider range of options are available for material selection.

The closure device may be used in a variety of applications requiring a fluid seal which can be readily broken upon application of force - for instance, the closure device according to the invention may be employed in the sealing of drinks containers. Also, it can be used as a so-called bursting disc which ruptures by separation of the blocking portion from the rim portion in response to the application of load or pressure. It will be appreciated that the blocking portion need not necessarily be disc-shaped when used in a bursting disc-type device.

The closure device is particularly useful in a fluid handling device, e.g. a syringe, as described in EP-A-776225.

Thus, according to a second aspect of the present invention, there is provided a fluid handling device having a hollow needle for dispensing fluid and a needle retraction assembly for transferring the needle after use into a housing of the device, the housing being provided with a closure device as defined above, the aperture defined by the rim portion constituting a needle entry aperture which, prior to operation of the needle retraction assembly, is closed by the blocking portion, the blocking portion being broken away during needle retraction to allow passage of the needle into the housing via said opening.

The sealing effected at said interface serves to prevent ingress or egress of fluid into the housing from a fluid holding chamber of the fluid handling device.

The closure device may be fitted to the housing or alternatively the rim portion of the closure device may be integrally connected with the housing.

The housing preferably forms a plunger or piston received within a tubular body portion of the fluid handling device which acts as a fluid-holding reservoir in use, and the blocking portion when held captive within the aperture may project forwardly of the remainder of the housing for co-operation with part of the needle retraction mechanism.

The invention will now be described by way of example only with reference to the accompanying drawings in which:
Figure 1 is a view of a syringe in accordance with the invention in its charged condition prior to use;
Figure 2 is a fragementary sectional view on an enlarged scale illustrating the relative positioning of a piston and body portion of the syringe part-way towards completion of the dispensing action;
Figure 3 is a diagrammatic view showing a modified zone of contact between the rim and blocking portions; and
Figure 4 is a diagrammatic view of a disc-type closure assembly.

The disposable syringe shown in the drawings may be generally similar to that described and illustrated in our prior EP-A-776225. Specifically, apart from the design of the leading end of the piston, the needle retraction mechanism is essentially the same as that described in EP-A-776225 and reference should therefore be made to EP-A-776225 for a detailed description of the syringe and its operation.

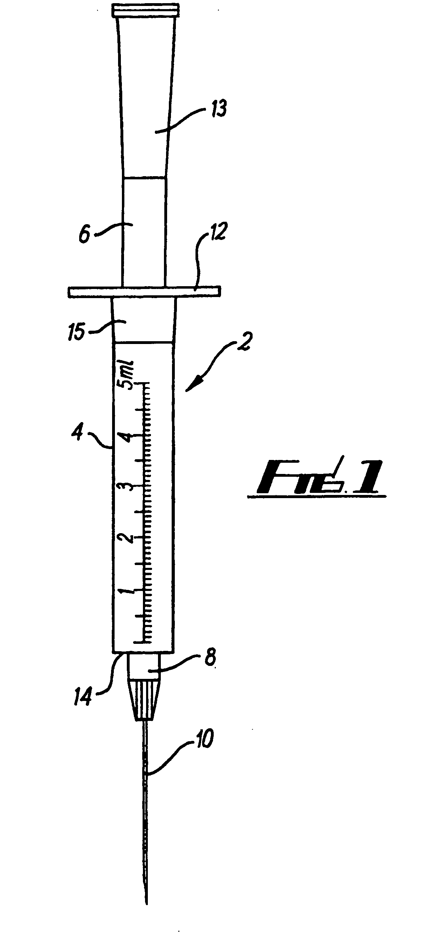

Referring now to the drawings, the disposable syringe 2 comprises a hollow body portion 4, from the rear of which (upper end as viewed in Figure 1) protrudes a piston 6. At the lower end of the body portion 4 is provided a mounting portion 8 for a needle 10 having a through passage and at the inner end of which is a hut 18.

The piston 6 is hollow having its trailing end closed by means of a cap (not shown). The forward end of the piston is arranged to receive a closure device or assembly 42 which engages firmly in the piston to form an end wall of the piston while defining an aperture 44 which is sufficiently large to allow the needle and hub 18 to pass through during needle retraction. The device 42 comprises a rim portion 45 fitted with a forwardly projecting blocking portion 46 of generally frusto-conical configuration so that the blocking portion 46 closes the aperture 44. The blocking portion 46 and rim portion 45 seal the forward end of the piston to prevent ingress of liquid into the piston interior from the interior of body portion 4.

The blocking portion 46, by virtue of moulding, becomes integrated with, and hence captive with, the rim portion 45. To this end, in the illustrated embodiment the blocking portion is formed with an annular flange 48 having a generally frusto-conical configuration surface 50 which is received within a complementary annular rebate 52 formed in the internal wall of the rim portion 45 thereby forming a detent. The mating conical surfaces of the flange 48 and the rebate 52 converge towards an imaginary apex in the direction of travel of the needle into the interior of the piston during needle retraction as described below. The interengagement between the flange 48 and the rebate 52 renders the blocking portion 46 axially captive to the rim portion 45 with the forward end of blocking portion 46 projecting forwardly of the piston for eventual engagement with the extension 38 of the hub 18. Also, the mating surfaces of the flange 48 and the rebate 52 form a zone of contact between the rim portion 45 and the blocking portion 46 to provide the above-mentioned seal between the blocking portion 46 and the aperture 44.

In operation, when the piston has been substantially fully depressed, the blocking portion 46 is subjected to loading as a result of contact with the extension 38 and, by appropriate design, when the load reaches a predetermined magnitude, the force on the blocking portion 46 becomes sufficient to dislodge the flange 48 from the rebate 52 so that the blocking portion together with the assembly comprising the needle 10 and the end portion 18 are automatically propelled by spring 28 into the interior of the piston 6, e.g. to avoid a needle stick hazard. Release of the flange 48 from the rebate 52 may be facilitated by resilient deformation of the rim portion and/or blocking portion if these components are produced from resiliently deformable plastics materials such as polyethylene or polypropylene.

The closure device comprising parts 45 and 46 is conveniently produced in the course of a two-shot moulding process, for instance a first stage in which the blocking portion is produced followed by a second stage in which the rim portion 45 is moulded onto the blocking portion thereby connecting the two parts together through the interengaging flange and rebate and providing a zone of intimate contact for sealing the aperture 44.

To prevent the parts 45 and 46 welding together inseparably during the moulding process, the two parts may be moulded using materials which are compatible with each other and have different characteristics. However, as previously indicated, some degree of fusion bonding between the rim and closure parts may be desirable, e.g. to ensure sealing and, where desired, to play a role in predetermining the loading necessary to break the blocking portion away from the rim portion.

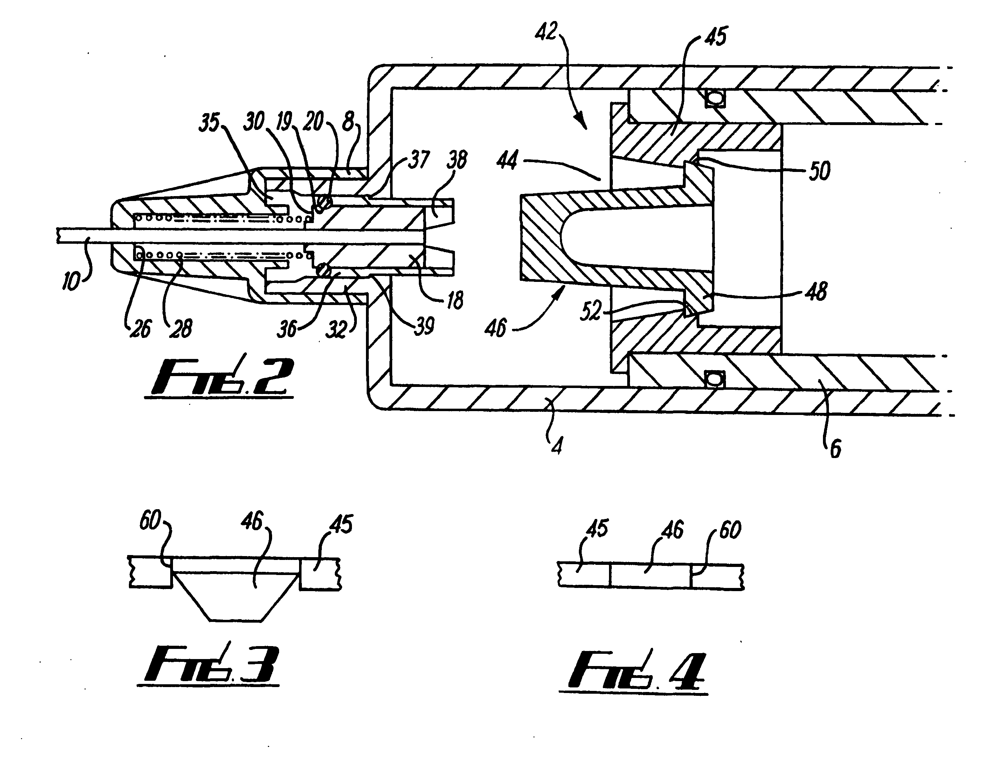

The closure device in Figures 1 and 2 is shown fitted into the forward end of the piston 6; however, in an alternative embodiment, the rim portion may be formed integrally with at least the forward end portion of the piston so that the piston and rim portion can be produced as a single moulding. For example, both the piston and rim portion may be made from a plastics material such as polyethylene or polypropylene.

In the embodiment of Figures 1 and 2, the blocking portion is held captive by the detent arrangement. However, the detent arrangement may be dispensed with, or it may be supplemented, by resistance developed at the zone of sealing contact, i.e. the moulding interface, between the blocking portion and the rim portion. Figure 3 illustrates a modification in which blocking portion is rendered captive to the rim portion by virtue of an interference or shrink type fit between the parts at a zone of contact 60 and without the aid of a detent arrangement. As previously mentioned, the shrink fit may be obtained during the moulding process by moulding the rim portion 45 around the blocking portion 46 and exploiting material shrinkage on cooling to secure the interference fit. Where the blocking portion is held captive in this way, there is not necessarily any signficant fusion bonding between the materials although, if desired, the material(s) may be selected so that such fusion bonding is present, e.g. as a result of some degree of diffusion of material between the two parts.

Figure 4 illustrates diagrammatically a closure device with a plate-like or disc-like configuration in which the trailing and forward surfaces of the rim portion 45 and the blocking portion 46 are substantially flush with each other. In the embodiment of Figure 4, the blocking portion 46 is rendered captive to the rim portion 45 at the zone of contact 60 by the shrinkage and/or fusion bonding mechanisms described above. However, the blocking portion may instead or additionally be held captive by a detent arrangement in the vicinity of the zone of contact 60.

Because the closure device of the present invention does not use an integral knock-out section as in EP-A-776225 to create the needle entry aperture, it will be seen that the closure is not limited to brittle materials such as styrene. Because of this increased flexibility, the possibility arises of making the entire safety syringe using materials such as polyethylene and/or polypropylene. The ability to call upon a wider range of fabrication materials may also be advantageous in terms of compatability with the drugs to be dispensed. The manner in which the blocking portion is held captive within the rim portion allows separation of the blocking portion by a predictable and controllable breaking force while securing a clean and dimensionally well-defined break-out aperture thus avoiding interference with needle travel during retraction.In each of the embodiments described above, it will be appreciated that the closure device functions as a bursting disc-type device and whilst, as illustrated in Figure 4, the blocking portion may be generally disc-shaped it may have other configurations as illustrated in Figures 2 and 4 for example.

## Claims

1. A device comprising a rim portion (45) defining an aperture and a removable blocking portion (46) which closes the aperture, said portions (45, 46) being united in sealing contact with each other at a moulding interface which extends around the blocking portion, both portions (45*,* 46) being produced as plastics mouldings, one portion having been produced in advance of the other so that moulding of the other portion can be effected with said one portion *in situ*, the blocking portion (46) being irreversibly removable from the rim portion (45) that, once the two portions have been separated from one another, they cannot be restored to the same condition of sealing and separation resistance simply by re-inserting the blocking portion (46) into the rim portion (45).

2. A device as claimed in Claim 1 in the form of a bursting disc arranged to rupture at a predetermined load determined at least in part, optionally, predominantly or substantially entirely, by the strength of fusion bonding between the blocking portion and the rim portion.

3. A device as claimed in Claims 1 or 2 in which the blocking portion and rim portion are coupled together by a detent arrangement (48, 52) at said interface.

4. A device as claimed in Claim 3 in which the resistance to separation afforded by the detent arrangement (48, 52) is supplemented by fusion bonding between the blocking portion and the rim portion.

5. A device as claimed in any one of Claims 1 to 4 in which the two portions (45, 46) are of the same plastics material.

6. A device as claimed in any one of Claims 1 to 5 in which the two portions (45, 46) are interlocked with each other so that some deformation of at least one of the parts occurs when breaking away the blocking portion.

7. A device as claimed in any one of Claims 1 to 6 in which the arrangement is such that the two portions (45, 46) are united together by the moulding process to afford a well-defined threshold at which the blocking portion breaks away from the rim portion in response to the application of mechanical force to the blocking portion.

8. A device as claimed in any one of Claims 1 to 7 in which the arrangement is such that the blocking portion breaks away cleanly from the rim portion at said interface to leave a dimensionally well-defined aperture.

9. A device as claimed in any one of Claims 1 to 8 in which one of the portions is produced as a plastics moulding in a mould having a moulding surface or surfaces defined, in part, by the other portion so as to provide said moulding interface, said one portion comprising the blocking portion.

10. A device as claimed in any one of Claims 1 to 9, in which both portions are produced as plastics mouldings in a two-shot moulding process.

11. A device as claimed in any one of Claims 1 to 4, or any one of Claims 6 to 10 when dependent on any one of Claims 1 to 4, in which the two portions comprise plastics materials having different chemical compositions and/or characteristics.

12. A device as claimed in any one of Claims 1 to 11 in which the blocking portion is capable of being broken away from the rim portion without deformation of either of the two portions.

13. A device as claimed in any one of Claims 1 to 12 in which, at the moulding interface, an annular surface of one of said portions is conformed with an annular surface of the other portion in the course of moulding said one portion.

14. A device as claimed in any one of Claims 1 to 13 in which, at the moulding interface, the portions intimately contact each other with at least sufficient fusion bonding therebetween to afford sealing engagement.

15. A device as claimed in any one of Claims 1 to 14 including means for resisting removal of the blocking portion so that the blocking portion can only be separated from the rim portion by application of axial load of predetermined magnitude.

16. A container sealed against fluid ingress or egress by a device according to any one of Claims 1 to 15.

17. A plunger (6) for a fluid handling device, the plunger being tubular and having one end thereof closed by a device according to any one of Claims 1 to 15.

18. A fluid handling device having a hollow needle (10) for dispensing fluid and a needle retraction assembly for transferring the needle after use into a housing (6) of the fluid handling device, the housing being provided with a device according to any one of Claims 1 to 15, the aperture being defined by the rim portion (45) constituting a needle entry aperture which, prior to operation of the needle retraction assembly, is closed by the blocking portion (46), the blocking portion being broken away from the rim portion (45) during needle retraction to allow passage of the needle (10) into the housing (6) via said opening.

19. A device as claimed in Claim 18 in which the rim portion is integral with the housing.

20. A device as claimed in Claim 19 in which the rim portion and housing both comprise polyethylene and/or polypropylene.

21. A method of producing a device as claimed in Claim 1, said method comprising producing one of the portions and moulding the other portion in the presence of said one portion portion using a surface of the latter to define a moulding boundary whereby the blocking portion seals the opening in the rim portion and can be broken away from the rim portion upon the application of a predetermined force to the blocking portion.

22. A method as claimed in Claim 21 in which said one portion comprises the blocking portion.

23. A method for accessing the interior of a hollow body or vessel provided with a device according to any one of Claims 1 to 15 comprising applying force to the blocking portion to break the same away from the rim portion.

## Patentansprüche

1. Vorrichtung, welche ein Randteil (45) umfasst, welches eine Öffnung und ein die Öffnung verschließendes lösbares Sperrteil (46) definiert, wobei diese Teile (45, 46) in einem Dichtkontakt an einer ausgeformten Berührungsfläche miteinander verbunden sind, welcher sich um das Sperrteil herum erstreckt, wobei beide Teile (45, 46) als Kunststoffformteil hergestellt sind, wobei ein Teil vor dem anderen Teil hergestellt wurde, so dass das Formen des anderen Teils mit dem ersten Teil an Ort und Stelle erfolgen kann, wobei das Sperrteil unwiederbringlich lösbar von dem Randteil (45) ist, dass, sobald die beiden Teile voneinandergetrennt wurden, diese ihren gleichen Dichtzustand und Trennwiderstand nicht wieder durch einfache Rückeinführung des Sperrteils (46) in das Randteil (45) herstellen können.

2. Vorrichtung nach Anspruch 1 in Form einer Berstscheibe, welche ausgebildet ist um bei einer vorbestimmten Belastung zu brechen, welche zumindest teilweise, optional ganz durch die Kraft einer Schmelz-Verbindung zwischen dem Sperrteil und dem Randteil bestimmt wird.

3. Vorrichtung nach Anspruch 1 oder 2, wobei das Sperrteil und das Randteil durch die Rastanordnung (48, 52) an der Berührungsfläche miteinander verbunden sind.

4. Vorrichtung nach Anspruch 3, wobei der Wiederstand zur Trennung, welche durch eine Rastanordnung (48, 52) gegeben ist, durch eine Schmelz-Verbindung zwischen dem Sperrteil und dem Randteil ergänzt ist.

5. Vorrichtung nach einem der Ansprüche 1 bis 4, wobei die beiden Teile (45, 46) aus gleichem Kunststoffmaterial sind.

6. Vorrichtung nach einem der Ansprüche 1 bis 5, wobei die beiden Teile (45, 46) ineinander greifen, so dass eine Verformung einer der beiden Teile entsteht, wenn das Sperrteil abgebrochen wird.

7. Vorrichtung nach einem der Ansprüche 1 bis 6, wobei die Anordnung derart ausgebildet ist, dass die beiden Teile (45, 46) mittels einem Formprozess miteinander verbunden sind, um einen eindeutigen Grenzwert zu definieren, bei welchem das Sperrteil von dem Randteil abbricht als Reaktion auf die Anwendung von mechanischen Kräften auf das Sperrteil.

8. Vorrichtung nach einem der Ansprüche 1 bis 7, wobei die Anordnung derart ist, dass das Sperrteil an der Berührungsfläche sauber von dem Randteil abbricht, um eine eindeutige formbeständige Öffnung zu hinterlassen.

9. Vorrichtung nach einem der Ansprüche 1 bis 8, wobei ein Teil als Kunststoffformteil in einer Form hergestellt ist, welche teilweise eine Formoberfläche oder Formoberflächen des anderen Teils definiert, so wie zur Bildung der ausgeformten Berührungsfläche, wobei das eine Teil das Sperrteil aufweist.

10. Vorrichtung nach einem der Ansprüche 1 bis 9, wobei die beiden Teile als Kunststoffformteile in einem zweistufigen Formprozess hergestellt sind.

11. Vorrichtung nach einem der Ansprüche 1 bis 4 oder nach einem der Ansprüche 6 bis 10, wenn diese abhängig nach einem der Ansprüche 1 bis 4 sind, wobei die beiden Teile ein Kunststoffmaterial aufweisen, welches unterschiedliche chemische Zusammensetzungen und/oder Eigenschaften aufweist.

12. Vorrichtung nach einem der Ansprüche 1 bis 11, wobei das Sperrteil geeignet ist, ohne eine Verformung eines der beiden Teile von dem Randteil abzubrechen.

13. Vorrichtung nach einem der Ansprüche 1 bis 12, wobei an dem Formanschluss eine ringförmige Oberfläche von einem der beiden Teile entspricht der ringförmigen Oberfläche des anderen Teils im Verlauf der Ausformung des ersten Teils.

14. Vorrichtung nach einem der Ansprüche 1 bis 13, wobei an der ausgeformten Berührungsfläche die Teile gegenseitig engen Kontakt mit mindestens einer dazwischen ausreichende Schmelz-Verbindung aufweisen, um eine Dichtwirkung zu ermöglichen.

15. Vorrichtung nach einem der Ansprüche 1 bis 14, welche Mittel gegen die Entfernung des Sperrteils umfasst, so dass das Sperrteil lediglich von dem Randteil mittels Anwendung einer axialen Belastung mit vorbestimmter Größe getrennt werden kann.

16. Gehäuse, welches durch eine Vorrichtung gemäß einem der Ansprüche 1 bis 15 gegen Flüssigkeitsein- oder austritt abgedichtet ist.

17. Ein Kolben (6) für eine Flüssigkeitsbedienungsvorrichtung, wobei der Kolben rohrförmig ist und ein geschlossenes Ende mittels einer Vorrichtung gemäß einem der Ansprüche 1 bis 15 ausbildet.

18. Eine Flüssigkeitsbedienungsvorrichtung, welche eine hohle Nadel (10) zur Verabreichung von Flüssigkeit aufweist und eine Rückzugvorrichtung für eine Nadel zur Überführung der Nadel nach Gebrauch in ein Gehäuse (6) der Flüssigkeitsbedienungsvorrichtung, wobei das Gehäuse mit einer Vorrichtung gemäß einem der Ansprüche 1 bis 15 ausgestattet ist und wobei die Öffnung durch das Randteil definiert ist, welche eine Eingangsöffnung für eine Nadel ausbildet, welche vor dem Betrieb der Rückzuganordnung der Nadel mittels dem Sperrteil (46) geschlossen ist, wobei das Sperrteil während des Nadelrückzuges von dem Randteil abbricht, um der Nadel den Durchgang in das Gehäuse (6) über dessen Eingang zu ermöglichen.

19. Vorrichtung nach Anspruch 18, wobei das Randteil ein Bestandteil des Gehäuses ist.

20. Vorrichtung nach Anspruch 19, wobei das Randteil sowie das Gehäuse Polyethylen und/ oder Polypropylen aufweisen.

21. Verfahren zur Herstellung einer Vorrichtung nach Anspruch 1, wobei das Verfahren die Herstellung eines Teils und die Formung des anderen Teils in Anwesenheit des Ersten Teils aufweist, Teilverwendung einer Oberfläche des vorbenannten Teils um eine Formungsbegrenzung zu definieren, wobei das Sperrteil die Öffnung in das Randteil abdichtet und von dem Randteil abbrechen kann unter Anwendung einer vorbestimmten Kraft auf das Sperrteil.

22. Verfahren nach Anspruch 21, wobei das erste Teil ein Sperrteil aufweist.

23. Verfahren zum Eintritt in das Innere eines hohlen Körpers oder Behälters, welcher mit einer Vorrichtung gemäß den Ansprüchen 1 bis 15 ausgestattet ist, wobei das Verfahren die Anwendung von Kraft auf das Sperrteil aufweist, um das Sperrteil von dem Randteil abzubrechen.

## Revendications

1. Dispositif comprenant une partie de bord (45) définissant une ouverture ainsi qu'une partie de blocage amovible (46) qui ferme l'ouverture, lesdites parties (45, 46) étant mutuellement réunies en contact de scellage au niveau d'une interface de moulage qui s'étend autour de la partie de blocage, les deux parties (45, 46) étant produites comme des moulages en plastique, une partie ayant été produite avant l'autre, de sorte que le moulage de l'autre partie puisse être effectué avec ladite première partie in situ, la partie de blocage (46) étant irréversiblement amovible depuis la partie de bord (45), de sorte qu'une fois que les deux parties ont été mutuellement séparées elles ne puissent pas être rétablies dans la même condition de scellage et de résistance à la séparation par simple réinsertion de la partie de blocage (46) dans la partie de bord (45).

2. Dispositif selon la revendication 1, ayant la forme d'un disque de rupture agencé pour se briser sous une charge prédéterminée, déterminée au moins en partie, optionnellement, de manière prédominante ou sensiblement complètement par la résistance du collage par fusion entre la partie de blocage et la partie de bord.

3. Dispositif selon la revendication 1 ou 2, dans lequel la partie de blocage et la partie de bord sont couplées l'une à l'autre par un agencement à encliquetage (48, 52) au niveau de ladite interface.

4. Dispositif selon la revendication 3, dans lequel la résistance à la séparation supportée par l'agencement à encliquetage (48, 52) est renforcée par collage par fusion entre la partie de blocage et la partie de bord.

5. Dispositif selon l'une quelconque des revendications 1 à 4, dans lequel les deux parties (45, 46) sont faites de la même matière plastique.

6. Dispositif selon l'une quelconque des revendications 1 à 5, dans lequel les deux parties (45, 46) sont emboîtées l'une avec l'autre de manière à ce qu'une certaine déformation d'au moins une des pièces ait lieu lors du détachement de la partie de blocage.

7. Dispositif selon l'une quelconque des revendications 1 à 6, dans lequel l'agencement est tel que les deux parties (45, 46) sont mutuellement réunies par le procédé de moulage pour fournir un seuil bien défini au niveau duquel la partie de blocage se détache de la partie de bord en réponse à l'application d'une force mécanique sur la partie de blocage.

8. Dispositif selon l'une quelconque des revendications 1 à 7, dans lequel l'agencement est tel que la partie de blocage.se détache de manière nette de la partie de bord au niveau de ladite interface pour laisser une ouverture bien définie au niveau des dimensions.

9. Dispositif selon l'une quelconque des revendications 1 à 8, dans lequel l'une des parties est produite comme un moulage en plastique dans un moule ayant une surface ou des surfaces de moulage, en partie définie(s) par l'autre partie, de manière à produire ladite interface de moulage, ladite première partie comprenant la partie de blocage.

10. Dispositif selon l'une quelconque des revendications 1 à 9, dans lequel les deux parties sont produites comme des moulages en plastique au cours d'un procédé de moulage à deux temps.

11. Dispositif selon l'une quelconque des revendications 1 à 4 ou selon l'une quelconque des revendications 6 à 10 en ce qu'elles dépendent de l'une quelconque des revendications 1 à 4, dans lequel les deux parties comprennent des matières plastiques ayant des compositions et/ou des caractéristiques chimiques différentes.

12. Dispositif selon l'une quelconque des revendications 1 à 11, dans lequel la partie de blocage peut être détachée de la partie de bord sans déformation d'aucune des deux parties.

13. Dispositif selon l'une quelconque des revendications 1 à 12, dans lequel, au niveau de l'interface de moulage, une surface annulaire d'une desdites parties est conformée sur une surface annulaire de l'autre partie au cours du moulage de ladite première partie.

14. Dispositif selon l'une quelconque des revendications 1 à 13, dans lequel, au niveau de l'interface de moulage, les parties entrent en contact mutuel intime au moins au moyen d'un collage par fusion suffisant entre celles-ci pour permettre une prise de scellage.

15. Dispositif selon l'une quelconque des revendications 1 à 14, comprenant des moyens destinés à résister au retrait de la partie de blocage de sorte que la partie de blocage ne puisse être séparée de la partie de bord que par application d'une charge axiale d'une grandeur prédéterminée.

16. Récipient rendu étanche à l'entrée ou à la sortie de fluide au moyen d'un dispositif selon l'une quelconque des revendications 1 à 15.

17. Piston (6) pour un dispositif de manipulation de fluides, le piston étant tubulaire et ayant une extrémité de celui-ci fermée par un dispositif selon l'une quelconque des revendications 1 à 15.

18. Dispositif de manipulation de fluides ayant une aiguille creuse (10) pour distribuer du fluide et un ensemble de rétraction d'aiguille pour transférer l'aiguille, après usage, dans un logement (6) du dispositif de manipulation de fluides, le logement étant muni d'un dispositif selon l'une quelconque des revendications 1 à 15, l'ouverture étant définie par la partie de bord (45) constituant une ouverture d'entrée d'aiguille qui est fermée, avant l'actionnement de l'ensemble de rétraction d'aiguille, par la partie de blocage (46), la partie de blocage étant détachée de la partie de bord (45) au cours de la rétraction de l'aiguille pour permettre le passage de l'aiguille (10) dans le logement (6) par l'intermédiaire de ladite ouverture.

19. Dispositif selon la revendication 18, dans lequel la partie de bord est d'une seule pièce avec le logement.

20. Dispositif selon la revendication 19, dans lequel la partie de bord et le logement comprennent tous les deux du polyéthylène et/ou du polypropylène.

21. Procédé de production d'un dispositif selon la revendication 1, ledit procédé comprenant les étapes consistant à produire l'une des parties et mouler l'autre partie en présence de ladite première partie en utilisant une surface de cette dernière pour définir une frontière de moulage, moyennant quoi la partie de blocage scelle l'ouverture dans la partie de bord et elle peut être détachée de la partie de bord sur application d'une force prédéterminée sur la partie de blocage.

22. Procédé selon la revendication 21, dans lequel ladite une partie comprend la partie de blocage.

23. Procédé destiné à accéder à l'intérieur d'un corps ou récipient creux muni d'un dispositif selon l'une quelconque des revendications 1 à 15, comprenant l'application d'une force sur la partie de blocage pour détacher celle-ci de la partie de bord.
